# EUROPEAN PATENT APPLICATION

(11) **EP 4 327 783 A2**
(43) Date of publication of application: **28.02.2024**
(21) Application number: 23192512.4
(22) Date of filing: 21.08.2023
(51) Int. Cl.: A61F 2/30, A61F 2/38

(54) **TIBIAL PROSTHESIS WITH DISTAL FEATURES FOR NON-CEMENTED FIXATION**

(30) Priority: 22.08.2022 US 202263399911 P
(71) Applicant: Zimmer, Inc., Warsaw, IN 46580 (US)
(72) Inventor: TRISCHLER, Cory, Warsaw, IN 46580 (US); BRANSCOME, Douglas G., Fort Wayne, IN 46804 (US); BISEK, Aaron J., Elk River, MN 55330 (US)
(74) Representative: Taor, Simon Edward William

(57) **Abstract**

The techniques described herein relate to a tibial prosthesis for a knee arthroplasty including a baseplate (100) and a tibial keel (108). The baseplate includes a distal surface (104) sized and shaped to substantially cover a proximal resected surface of a tibia; a proximal surface (102) opposite the distal surface, the proximal surface having a lateral compartment (116) and a medial compartment (114) opposite the lateral compartment; a periphery (106) extending between the distal surface and the proximal surface; a first layer of porous material (152) forming at least a majority of the distal surface and extending to the periphery; and a second layer of nonporous or relatively less porous material (154) having a plurality of reference features (124A-F) extending through the first layer, wherein the plurality of reference features form at least a portion of the distal surface; and a tibial keel extending distally from the distal surface to define a longitudinal tibial keel axis (S1).

## Description

### FIELD

The present subject matter relates to orthopedic prostheses and, more particularly, to tibial prostheses such as baseplates used in knee arthroplasties.

### BACKGROUND

Orthopedic procedures and prostheses are commonly utilized to repair and/or replace damaged bone and tissue in the human body. For example, a knee arthroplasty can be used to restore natural knee function by repairing damaged or diseased articular surfaces of the femur and/or tibia. An incision is made into the knee joint to expose the bones comprising the j oint. Cut guides are used to guide the removal of the articular surfaces that are to be replaced. Prostheses are used to replicate the articular surfaces. Knee prostheses can include a femoral component implanted on the distal end of the femur, which articulates with a tibial bearing component and a tibial component (sometimes called a tibial tray or tibial baseplate) implanted on the proximal end of a tibia. Together these components replicate the function of a healthy natural knee. Various types of arthroplasties are known including a total knee arthroplasty, where all of the articulating compartments of the joint are repaired with prosthetic components.

### OVERVIEW

This disclosure pertains generally to tibial prostheses used in a knee arthroplasty including a total knee arthroplasty. The present inventors have recognized, among other things, a tibial baseplate including distal features that can facilitate better initial fixation to bone and better bonding via bone ingrowth or on-growth such that the tibial baseplate can be fixedly retained on the proximal tibia. Better bonding can reduce micro-motion of the tibial baseplate and can provide better overall durability for the tibial baseplate. Furthermore, present inventors have recognized the distal features can provide for a greater rigidity and torsional strength for the tibial baseplate. Additionally, the present inventors contemplate apparatuses and systems that allow a surgeon to prepare the tibia during surgery but allow the surgeon to choose intraoperatively between a cemented apparatus or a non-cemented apparatus. In particular, the cemented apparatus and the non-cemented apparatus can share a same or similar stock size and can share a same or similar tibial keel and/or fin geometry (e.g., a substantially same distal profile, shape, size, etc. in two or more dimensions). Systems of different stock sizes of the cemented apparatuses and different stock sizes of the non-cemented apparatuses are provided herein. However, these can share substantially a same geometry according to a stock size. As an example, the surgeon has the option of intraoperatively switching from a non-cemented apparatus of a first stock size to a cemented apparatus of the same first stock size. This can reduce surgical complexity and time.

Additional features and benefits of the various examples provided herein will be discussed and/or will be apparent to one of ordinary skill in the art.

To further illustrate the apparatuses, systems and methods disclosed herein, the following non-limiting examples are provided, and which are referred to below as techniques. Parts or all of these examples/techniques can be combined in any manner.

In some aspects, the techniques described herein relate to a tibial prosthesis for a knee arthroplasty optionally including a baseplate and a tibial keel. The baseplate optionally including: a distal surface sized and shaped to substantially cover a proximal resected surface of a tibia; a proximal surface opposite the distal surface, the proximal surface having a lateral compartment and a medial compartment opposite the lateral compartment; a periphery extending between the distal surface and the proximal surface; a first layer of porous material forming at least a majority of the distal surface and extending to the periphery; and a second layer of non-porous or relatively less porous material having a plurality of reference features extending through the first layer, wherein the plurality of reference features form at least a portion of the distal surface; and a tibial keel extending distally from the distal surface to define a longitudinal tibial keel axis.

In some aspects, the techniques described herein relate to a tibial prosthesis such as described previously optionally including, further a plurality of fins spanning a junction between the tibial keel and the distal surface.

In some aspects, the techniques described herein relate to a tibial prosthesis such as described previously optionally including, wherein the plurality of fins are angled anterior-posterior and medial-lateral to form a flute on a posterior of the tibial keel.

In some aspects, the techniques described herein relate to a tibial prosthesis such as described previously optionally including, wherein the tibial keel includes a second flute and a third flute each extending to a distal tip of the tibial keel, wherein the second flute and the third flute are connected by a anterior fin on an anterior of the tibial keel.

In some aspects, the techniques described herein relate to a tibial prosthesis such as described previously optionally including, further including a plurality of pegs extending distally from the distal surface, wherein one or both of the plurality of pegs or the plurality of fins has a window formed of the porous material therein.

In some aspects, the techniques described herein relate to a tibial prosthesis such as described previously optionally including, wherein the plurality of pegs includes four pegs located adjacent, respectively, a posterior-medial corner, a posterior-lateral corner, an anterior-lateral corner and an anterior-medial corner of the periphery.

In some aspects, the techniques described herein relate to a tibial prosthesis such as described previously optionally including, wherein the plurality of pegs are one of square shaped or rectangular in cross-section and are configured to be press-fit into a round hole drilled into the tibia.

In some aspects, the techniques described herein relate to a tibial prosthesis such as described previously optionally including, wherein the window extends entirely through at least one of the plurality of fins.

In some aspects, the techniques described herein relate to a tibial prosthesis such as described previously optionally including, wherein one or more surfaces of at least one of the tibial keel or the plurality of fins is fabricated to be smoother than the distal surface to discourage bone ingrowth.

In some aspects, the techniques described herein relate to a tibial prosthesis system optionally including: a plurality of prostheses, each of the plurality of prostheses optionally including: a baseplate including: a distal surface sized and shaped to substantially cover a proximal resected surface of a tibia; a proximal surface opposite the distal surface, the proximal surface having a lateral compartment and a medial compartment opposite the lateral compartment; a periphery extending between the distal surface and the proximal surface; and a plurality of pegs extending distally from the distal surface; wherein a size of each of the plurality of prostheses differ with respect to the distal surface, the proximal surface and the periphery; and a tibial keel extending distally from the distal surface to a dome shaped distal tip, wherein the tibial keel has an elongate length as measured proximal-distal and defines a longitudinal tibial keel axis extending along the elongate length, wherein the elongate length increases in a step-wise manner with an increase in the size the plurality of prostheses with respect to the distal surface, the proximal surface and the periphery.

In some aspects, the techniques described herein relate to a system such as described previously optionally including, further a plurality of fins spanning a junction between the tibial keel and the distal surface.

In some aspects, the techniques described herein relate to a system such as described previously optionally including, further including: a first layer of porous material forming at least a majority of the distal surface and extending to the periphery; and a second layer of non-porous or relatively less porous material forming a base; wherein the plurality of pegs are formed of both the porous material and the non-porous or relatively less porous material.

In some aspects, the techniques described herein relate to a system such as described previously optionally including, wherein the tibial keel includes a first flute on a posterior side formed between the plurality of fins in addition to the tibial keel, and wherein the tibial keel includes a second flute and a third flute each extending to a distal tip of the tibial keel, wherein the second flute and the third flute are connected by a anterior fin on an anterior of the tibial keel.

In some aspects, the techniques described herein relate to a system such as described previously optionally including, wherein the second layer of the non-porous or relatively less porous material has a plurality of reference features extending through the first layer, and wherein the plurality of reference features form at least a portion of the distal surface.

In some aspects, the techniques described herein relate to a system such as described previously optionally including, wherein one or more surfaces of at least one of the tibial keel or the plurality of fins is fabricated to be smoother than the distal surface to discourage bone ingrowth.

In some aspects, the techniques described herein relate to a tibial prosthesis system optionally including: a first tibial prosthesis optionally including: a first proximal surface; a first distal surface opposite the first proximal surface, the first distal surface sized and shaped to substantially cover a proximal resected surface of a tibia; at least one pocket formed in the first tibial prosthesis and recessed from the first distal surface, wherein the at least pocket is configured to receive a bone cement therein; a first keel extending distally from the first distal surface; and a first plurality of fins spanning a junction between the first keel and the first distal surface; and a second tibial prosthesis optionally including: a second proximal surface; a second distal surface opposite the second proximal surface, the second distal surface sized and shaped to substantially cover the proximal resected surface of the tibia; a plurality of pegs extending distally from the second distal surface; a first layer of porous material forming at least a majority of the second distal surface; a second layer of nonporous material forming at least a portion of the second tibial prosthesis; a second keel extending distally from the second distal surface; and a second plurality of fins spanning a junction between the second keel and the second distal surface; wherein the first keel and first plurality of fins share a substantially same geometry as the second keel and the second plurality of fins.

In some aspects, the techniques described herein relate to a system such as described previously optionally including, wherein the substantially same geometry includes at least two of: an elongate length as measured proximal-distal, a distal end profile, and a medial-lateral width.

In some aspects, the techniques described herein relate to a system such as described previously optionally including, wherein the first plurality of fins are angled anterior-posterior and medial-lateral to form a first flute on a posterior of the first tibial keel, wherein the second plurality of fins are angled anterior-posterior and medial-lateral to form a first flute on a posterior of the second tibial keel, and wherein the first flute of the first tibial prosthesis and the first flute of the second tibial prosthesis have substantially a same angle.

In some aspects, the techniques described herein relate to a system such as described previously optionally including, wherein the first plurality of fins are relatively thinner than the second plurality of fins.

In some aspects, the techniques described herein relate to a system such as described previously optionally including, wherein the first tibial keel includes a second flute and a third flute each extending to a distal tip of the first tibial keel, wherein the second tibial keel includes a second flute and a third flute each extending to a distal tip of the tibial keel, wherein the second flute and the third flute of the first tibial keel share substantially a same geometry as the second flute and the third flute of the second tibial keel.

### BRIEF DESCRIPTION OF THE DRAWINGS

In the drawings, which are not necessarily drawn to scale, like numerals can describe similar components in different views. Like numerals having different letter suffixes can represent different instances of similar components. The drawings illustrate generally, by way of example, but not by way of limitation, various examples discussed in the present document.
FIGS. 1A-1E are plan views of a tibial baseplate from various perspectives according to an example of the present application.
FIGS. 1F-1L are various enlarged and/or cross-sectional views of the tibial baseplate of FIGS. 1A-1E according to an example of the present application.
FIG. 2 illustrates a system including a plurality of tibial baseplates with keels of different proximal-distal lengths according to an example of the present application.
FIG. 2A is a chart of changes in the proximal-distal lengths of the keel as compared with a standard size of the tibial baseplate according to an example of the present application.
FIGS. 3A-8B shows a system that provides for various sizes of tibial baseplates including those configured for both non-cemented and cemented fixation to the tibia, the tibial baseplates can have a same standard size (both non-cemented and cemented versions) and can share a substantially same distal profile with respect to the keel and plurality of fins according to an example of the present application.

### DETAILED DESCRIPTION

The present application relates to tibial prostheses, in particular, tibial baseplates and systems. This application focuses on distal aspects and features of the tibial baseplate as further discussed herein. As discussed previously, these distal features can improve fixation and the durability of the tibial baseplate among other benefits.

As used herein, the terms "proximal" and "distal" should be given their generally understood anatomical interpretation. The term "proximal" refers to a direction generally toward the torso of a patient, and "distal" refers to the opposite direction of proximal, i.e., away from the torso of a patient. It should be understood that the use of the terms "proximal" and "distal" should be interpreted as though the patient were standing with the knee joint in extension despite the apparatuses described herein generally being used with the knee joint in flexion. The intent is to differentiate the terms "proximal" and "distal" from the terms "anterior" and "posterior". As used herein, the terms "anterior" and "posterior" should be given their generally understood anatomical interpretation. Thus, "posterior" refers to a rear of the patient, e.g., a back of the knee. Similarly, "anterior" refers to a front of the patient, e.g., a front of the knee. Thus, "posterior" refers to the opposite direction of "anterior". Similarly, the term "lateral" refers to the opposite direction of "medial". The term "medial-lateral" means medial to lateral or lateral to medial. The term "proximal-distal" means proximal to distal or distal to proximal. The term "anterior-posterior" means anterior to posterior or posterior to anterior.

As used herein, the "periphery" of a tibial prosthesis refers to any periphery as viewed in a top plan view, e.g., in a generally transverse anatomical plane. Alternatively, the periphery of a tibial prosthesis may be any periphery as viewed in bottom plan view, e.g., in a generally transverse plane and looking at the distal surface adapted to contact a resected proximal surface of a tibial bone. In the context of a prosthesis, such as tibial baseplate described below, "home axis" refers to an axis oriented with respect to baseplate such that the baseplate home axis of baseplate is aligned with a home axis of tibia after implantation of baseplate in a proper rotational and spatial orientation. With some baseplate designs including the one illustrated herein, the home axis bisects a PCL cutout at the posterior edge of periphery of tibial plateau, and bisects anterior edge at the anterior edge of periphery of tibial plateau. It is contemplated that home axis may be oriented to other baseplate features, it being understood home axis of baseplate is positioned such that that proper alignment and orientation of baseplate upon tibia positions the home axis of baseplate coincident with home axis of tibia.

Home axis of tibial baseplate can be an anteroposterior axis, as the home axis extends generally anteriorly and posteriorly when baseplate is implanted upon tibia. Tibial baseplate also defines mediolateral axis, which lies along the longest line segment contained within periphery that is also perpendicular to home axis of baseplate. As described below, the home axis and mediolateral axis cooperate to define a coordinate system useful for quantifying certain baseplate features in accordance with the present disclosure. Additionally, distal features such as the keel and the one or more fins can be aligned with respect to the anteroposterior axis, the mediolateral axis or other axes such as an axis of symmetry, a proximal-distal axis, etc.

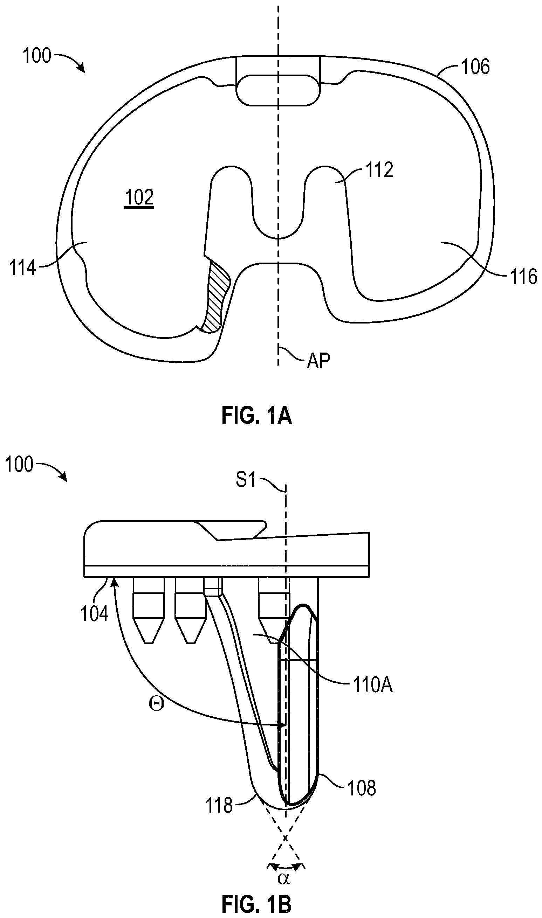

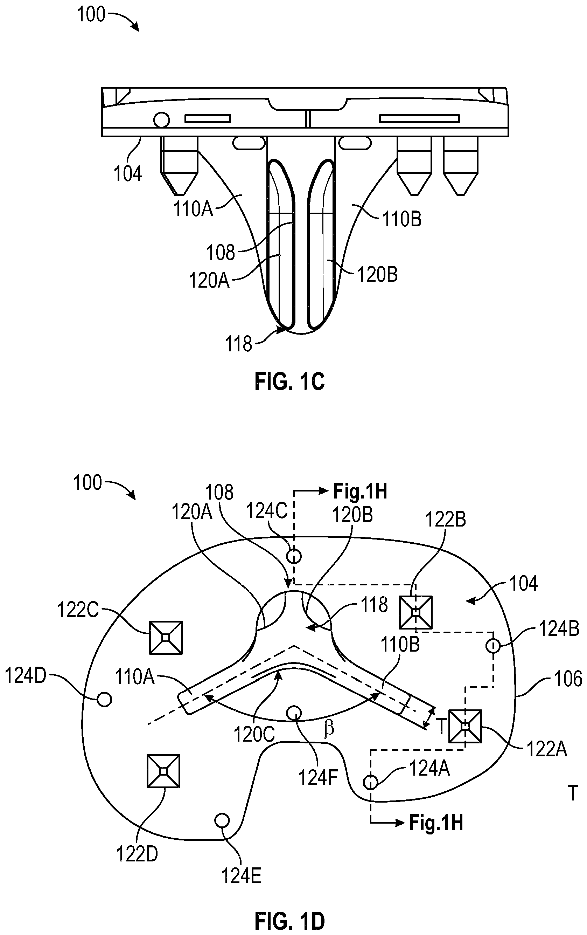

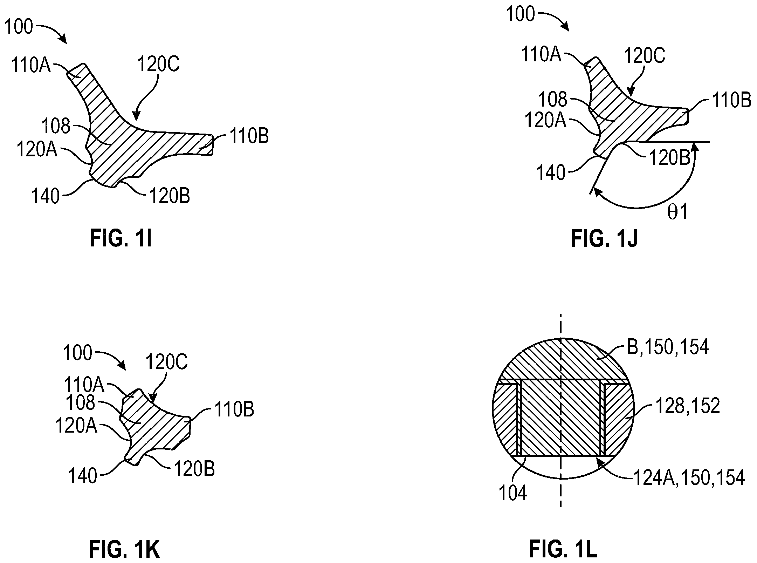

FIGS. 1A-1E show plan views of various sides of a tibial baseplate 100. The tibial baseplate 100 can include a proximal surface 102, a distal surface 104, a periphery 106. As shown in FIGS. 1B-1E the tibial baseplate 100 can include a keel 108, and a plurality of fins 110A and 110B (both shown in FIG. 1D).

FIG. 1A is a plan view of a proximal side of the tibial baseplate 100 showing the proximal surface 102, anteroposterior axis AP and periphery 106. Coupling features 112 such as bosses, rails, notches, and/or other features for coupling with a tibial bearing component (not shown) can be utilized on the proximal side. The coupling features 112 construction can be similar to that of the commercially available Persona^{®} Total Knee System from Zimmer Biomet Inc. of Warsaw, Indiana.

Tibial baseplate 100 can have a particular asymmetry, with respect to home axis and anteroposterior axis AP. This shape is designed to maximize tibial coverage for a large proportion of knee-replacement candidates as discussed in various of the Applicant's previously filed applications including United States Patent Application Publication Nos. 2013/0024001A1 and 2013/0131820A1, the entire specification of each of which is incorporated by reference in its entirety. Maximized coverage of cortical bone facilitates superior support of tibial baseplate 100. A firm, enduring fixation of tibial baseplate 100 to tibia is facilitated by large-area contact between the cortical and cancellous bone of tibia.

The anteroposterior axis AP can divide the tibial baseplate 100 into a medial compartment 114 and a lateral compartment 116. The medial compartment 114 can be asymmetrically shaped and sized with respect to the lateral compartment 116 as defined along the periphery 106. The periphery 106 can be shaped as a wall and can have the coupling features 112 there along at one or more select locations.

The tibial baseplate 100 can be available in a plurality of standard sizes. These standard sizes are selected to provide coverage to various sizes of tibia. As an example, the tibial baseplate 100 can include nine sizes from a size 1 (small stature) to a size 9 (large stature). The various sizes can be available as a system to a surgeon to make a proper size election. The standard sizes have different geometries including along the proximal side (e.g., periphery 106, proximal surface 102, etc.) and distal side (e.g., distal surface 104, keel 108, and the plurality of fins 110A and 110B, etc.) as further described herein.

FIGS. 1B-1E show further plan views of the tibial baseplate 100 from various sides. FIG. 1B shows the keel 108 and one of the plurality of fins 110A. The fin 110A can extend between the keel 108 and the distal surface 104. The keel 108 can extend generally distally from the distal surface 104 and remainder of the tibial baseplate 100. The fins 110A and/or 110B can be part of the keel 108 assembly but are described herein as distinct features from a remainder of the keel 108 that includes a stem feature. The keel 108 can be at an angle Θ with regard to the distal surface 104 (as measured from the distal surface 104 to an axis of symmetry S1 for the keel 108). According to the example of FIG. 1B, the angle Θ can be substantially 90 degrees. The axis of symmetry S1 of the keel 108 can be a proximal-distal axis.

FIG. 1B shows the keel 108 can include the stem feature having a distal tip 118. This distal tip 118 can be shaped for insertion into a canal of the tibia. Thus, the distal tip 118 can be shaped as a dome or hemisphere or otherwise shaped to lack sharp edges, for example. The surfaces that form the distal tip 118 can have a geometry to form an angle α of between 30 degrees and 70 degrees (substantially 50 degrees in some examples). The surfaces that form the distal tip 118 can be curved and can have a radius of curvature of between 3 mm and 6 mm, for example. According to one example the radius of curvature can be substantially 4.5 mm, for example. The distal tip 118 can include an apex, for example. It should be noted that the keel 108 with the distal tip 118 is not configured for coupling with a stem extension or other feature as is common in some total knee systems. Rather, the keel 108 generally increases in proximal-distal length with increases in the standard size of the tibial baseplate 100.

FIGS. 1C and 1D show the keel 108 and the plurality of fins 110A and 110B extending from the distal surface 104. The fins 110A and 110B can have a symmetric arrangement and mirror geometry with respect to one another and with respect to the keel 108 arranged upon opposing medial and lateral sides thereof. Although two fins 110A and 110B are shown in FIGS. 1C and 1D, other examples contemplate three or more fins. The keel 108 can include a first groove comprising a first flute 120A and a second groove comprising a second flute 120B. The first flute 120A and the second flute 120B can extend from a few millimeters from the distal surface 104 to the distal tip 118. Thus, the first flute 120A and the second flute 120B can extend a majority of the proximal-distal length of the keel 108 (e.g., from 50% of the length to 95%). The first flute 120A and the second flute 120B can be generally positioned on an anterior side of the keel 108 so as to be anterior facing. The first flute 120A and the second flute 120B can be symmetrically shaped with respect to one another. The geometry of the first flute 120A and the second flute 120B will be further discussed subsequently.

The keel 108 and the fins 110A and 110B can additionally form a third flute 120C (FIG. 1D) or recessed area along a posterior side of the keel 108 and the fins 110A and 110B. The third flute 120C can be primarily formed by the fins 110A and 110B, which can be angled relative to one another to form an angle β. In particular, the fins 110A and 110B can extend not only proximal-distal (as keel 108 in some examples) but can additionally extend medial-lateral and anterior-posterior. The angle β can be between substantially 105 degrees and 140 degrees, for example. According to one example, the angle β can be substantially 126 degrees. The fins 110A, 110B can have a radius of curvature relative to the keel 108 along the posterior side thereof. This radius of curvature can be between substantially 5 mm and 10 mm. According to one example, the radius of curvature can be substantially 7.6 mm. A thickness T (FIG. 1D) of the fins 110A and 110B can vary as desired but can be relatively thicker for a same stock size of the non-cemented prosthesis as compared with the cemented prosthesis. It should be noted that the thickness T changes moving proximal-distal along the fins 110A and 110B. However, the thickness T for the same stock size of the non-cemented prosthesis can be relatively thicker as compared with the thickness of the cemented prosthesis at the same proximal-distal position.

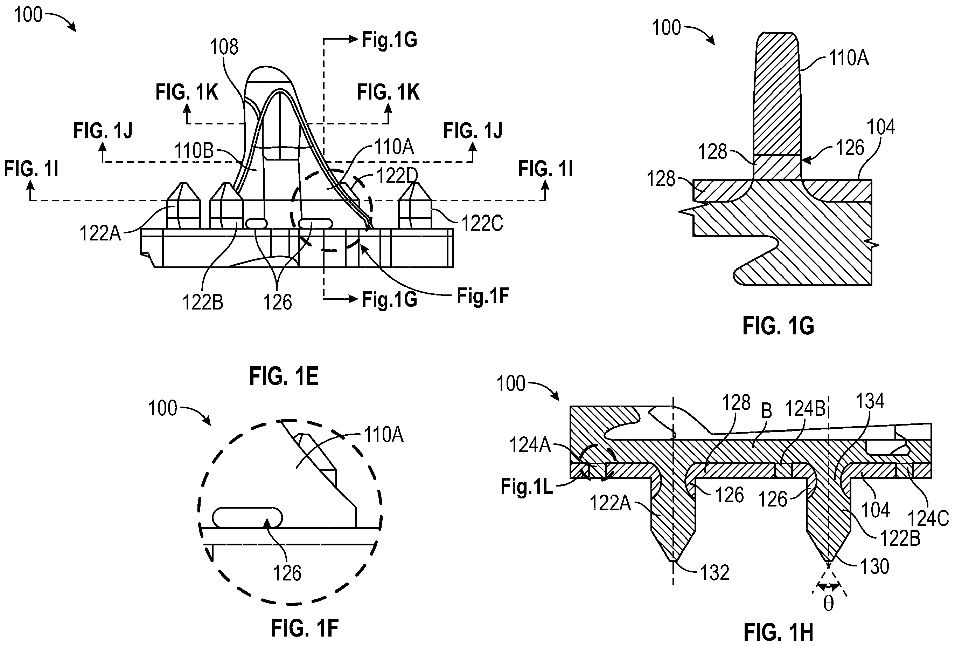

Distal surface 104 and other features such as the windows 126 of FIGS. 1E to 1G will be described subsequently. These features can be made of a porous or highly porous material that facilitates an amount of bone ingrowth. For example, the distal surface and windows 126 may also be constructed of, or may be coated with, a highly porous biomaterial. A highly porous biomaterial is useful as a bone substitute and as cell and tissue receptive material. A highly porous biomaterial may have a porosity as low as 30%, 55%, or as high as 70%, 80%, 85%, or 90%. The highly porous material can have an average pore size of between 100 microns and 1000 microns, for example.

An example of such porous or highly porous material is OsseoTi^{®} generally available from Zimmer Biomet, Inc., of Warsaw, Ind. The material can include titanium or titanium alloy and can additionally include other materials. Such material (including a base of relatively less porous or non-porous biocompatible material) can be manufactured using additive manufacturing processes such as laser sintering or the like. OsseoTi^{®} is highly biocompatible, has high corrosion resistance and includes a highly interconnected porous architecture that mimics the porous structure of human cancellous bone, which can enhance bone integration and in-growth. The porous or highly porous material can be manufactured to be layered over or otherwise structured with/on a relatively less porous or non-porous biocompatible material such as titanium, titanium alloy, stainless steel or other material as known in the art.

Another example of such a porous or highly porous material is produced using Trabecular Metal^{™} Technology generally available from Zimmer Biomet, Inc., of Warsaw, Ind. Such a material may be formed from a reticulated vitreous carbon foam substrate which is infiltrated and coated with a biocompatible metal, such as tantalum, by a chemical vapor deposition ("CVD") process in the manner disclosed in detail in U.S. Pat. No. 5,282,861 to Kaplan, the entire disclosure of which is hereby expressly incorporated herein by reference. In addition to tantalum, other metals such as niobium, or alloys of tantalum and niobium with one another or with other metals may also be used. The porous tantalum structure may be made in a variety of densities in order to selectively tailor the structure for particular applications. In particular, as discussed in the above-incorporated U.S. Pat. No. 5,282,861, the porous tantalum may be fabricated to virtually any desired porosity and pore size, and can thus be matched with the surrounding natural bone in order to provide an improved matrix for bone ingrowth and mineralization.

Generally, the porous material structures contemplated can include a large plurality of ligaments defining open spaces there between, with each ligament generally including a core covered by a thin film of metal. The open spaces between the ligaments form a matrix of continuous channels having no dead ends, such that growth of cancellous bone through the porous tantalum structure is uninhibited. The porous or highly porous material may include up to 70%, 85%, or more void space therein. Thus, porous or highly porous material is a lightweight, strong porous structure which is substantially uniform and consistent in composition, and closely resembles the structure of natural cancellous bone, thereby providing a matrix into which cancellous bone may grow to provide fixation of the tibial baseplate 100 to the patient's bone.

Referring to FIG. 1D, various distal features such as pegs 122A, 122B, 122C and 122D and reference features 124A, 124B, 124C, 124D, 124E and 124F are shown. The pegs 122A, 122B, 122C and 122D can have a square or rectangular shape. A width dimension of the pegs 122A, 122B, 122C and 122D can be on the order of 2.5 mm to 7.5 mm. A proximal-distal length (depth) of the pegs 122A, 122B, 122C and 122D can be between 5 mm and 15 mm, for example. The square or rectangular shaped pegs 122A, 122B, 122C and 122D can be configured to be press-fit into round (cross-section) holes of similar or smaller diameter drilled in the tibia, for example.

According to one example, the proximal-distal length (depth) of the pegs 122A, 122B, 122C and 122D can be between 7.5 mm and 12.5 mm. The pegs 122A, 122B, 122C and 122D can be asymmetrically arranged relative to the keel 108, the fins 110A, 110B, the periphery 106, the anteroposterior axis AP (FIG. 1A) and/or mediolateral axis. The pegs 122A, 122B, 122C and 122D can be arranged within 15 mm or less of the periphery 106. In particular, the pegs 122A, 122B, 122C and 122D can be located adjacent (within 12 mm or less of) the anterior-medial, posterior-medial, posterior-lateral and anterior-lateral corners of the periphery 106, respectively. Similarly, the pegs 122A, 122B, 122C and 122D can be located within 10 mm or less of a respective one of the fins 110A and 110B. The pegs 122A, 122B, 122C and 122D can be orientated at substantially 90 degrees or another angle to the distal surface 104.

The reference features 124A, 124B, 124C, 124D, 124E and 124F can comprise circular, cylindrical or otherwise shaped features of the base of relatively less porous or non-porous biocompatible material. The reference features 124A, 124B, 124C, 124D, 124E and 124F project distally from the base (not shown in FIG. 1D but shown in FIG. 1L) and form at least a portion of the distal surface 104 of the tibial baseplate 100. The reference features 124A, 124B, 124C, 124D,124E and 124F can be relatively small, on the order of 0.5 mm to 2.0 mm in diameter. As at least a majority (and preferably much more) of the distal surface 104 can be formed of the porous or highly porous material, it can be hard to judge an overall flatness or angulation of the distal surface 104 with such material(s). Thus, the reference features 124A, 124B, 124C, 124D,124E and 124F formed of the relatively less porous or non-porous biocompatible material can be utilized. The reference features 124A, 124B, 124C, 124D, 124E and 124F can be placed at various locations and project to form the distal surface 104 at various locations. The reference features 124A, 124B, 124C, 124D, 124E and 124F can be measured at the various locations of the distal surface 104 to determine if the distal surface 104 has a desired overall flatness or angulation.

FIG. 1E shows a plan view of the tibial baseplate 100 from a posterior-medial or posterior-lateral side showing both the plurality of fins 110A and 110B. FIG. 1E also shows windows 126. The windows 126 can be formed in the fins 110A, 110B, keel 108 and/or pegs 122A, 122B, 122C and 122D, for example. The windows 126 can be formed of the porous or highly porous material similar or identical to at least a majority of the distal surface 104.

FIG. 1F is an enlarged view of a portion of the fin 110A showing one of the windows 126. The window 126 can have an elongate medial-lateral width relative to a proximal-distal depth. The medial-lateral width can be between 5 mm and 10 mm, for example. According to one example the medial-lateral width can be about 6 mm. According to one example, the proximal-distal depth can be 1.7 mm. An edge of the window 126 can be between about 3.5 mm and 6.5 mm from the distal edge of the fin 110A.

FIG. 1G shows the window 126 with the porous or highly porous material 128. The window 126 can extend entirely through and can form a portion of the fin 110A. The window 126 can be positioned adjacent (distal of) the distal surface 104 formed in the area shown by the porous or highly porous material 128. A majority of the fin 110A particularly along distal portions and distal surfaces thereof can be constructed of the relatively less porous or non-porous biocompatible material. Indeed, according to some examples the fins 110A, 110B (not shown in FIG. 1G) and the keel 108 (not shown in FIG. 1G) can be fabricated (e.g., as-printed, machined, dry blasted, etc.) to have a relatively smoother surface finish (e.g., less than 8.0 micron Ra) than a surface finish of the distal surface 104 or other portions of the tibial baseplate 100. This relatively smooth surface finish as compared with the distal surface 104 and windows 126 formed with the porous or highly porous material 128 can discourage bone ingrowth or on-growth on distal portions/surfaces of the fins 110A, 110B, the keel 108 and or the pegs. However, surface finishing of the fins 110A and/or 110B and/or the keel 108 is not contemplated in some embodiments.

FIG. 1H shows a cross-section through the tibial baseplate 100 including through the distal surface 104, the pegs 122A and 122B, the reference features 124A, 124B and 124C, the windows 126, the base B and the porous or highly porous material 128. The pegs 122A and 122B can be symmetrically shaped and can each have relatively a same size and shape. A distal end 130 of the pegs 122A and 122B can be pyramid shaped and angled at about 30 degrees to an axis of symmetry thereof. Thus, the pegs 122A and 122B can form an angle θ with respect to opposing sides of the distal end 130. The angle θ can be about 60 degrees for example. The distal end 130 can have a blunt tip 132 of less than 1.0 mm in a major dimension, for example.

As shown in the example of FIG. 1H, the porous or highly porous material 128 may not extend fully through the pegs 122A and 122B at the windows 126 but may cover a proximal base portion 134 of the pegs 122A and 122B to a depth of between 0.25 mm and 1.5 mm. A majority of the pegs 122A and 122B, particularly along distal portions, distal end 130 and distal surfaces thereof, can be constructed of the relatively less porous or non-porous biocompatible material.

FIGS. 1I-1K show cross-sections of the keel 108 and the plurality of fins 110A and 110B showing features such as the first flute 120A, the second flute 120B and the third flute 120C. As shown in FIGS. 1I-1K the keel and the plurality of fins 110A and 110B can taper from adjacent the distal surface to the distal tip. The first flute 120A and the second flute 120B can be separated by an anterior fin 140. The anterior fin 140 can form an anterior-most portion of the keel 108 and can have a substantially uniform width for at least a majority of the proximal-distal length of the keel 108. The width of the anterior fin 140 can be on the order of 1 mm to about 3 mm, for example for the majority of the longitudinal length thereof. The width of the anterior fin 140 can increase adjacent a proximal portion of the keel 108 as shown in FIG. 1I. The first flute 120A can have an angle θ1 of between about 100 degrees and about 140 degrees. According to one example, the angle θ1 can be about 117 degrees. Similarly, the second flute 120B can have the same angle θ1 of between about 100 degrees and about 140 degrees. According to one example, the angle θ1 can be about 117 degrees. Keel 108 may not have a taper but can be substantially un-tapered along at least a majority or more of a proximal-distal length thereof. The fins 110A and 110B can be tapered as previously discussed.

The fins 110A and 110B can be monolithically or integrally formed with the remainder of the tibial baseplate 100 including the distal surface 104 and the keel 108. Thus, the fins 110A and 110B can be features of the keel 108 as discussed above. However, it is contemplated that fins 110A and 110B may be separately attachable to other features of the tibial baseplate 100. Alternatively, the fins 110A and 110B can be monolithically formed as a single piece together and can be coupled with the keel 108 or the distal surface 104 separately. The keel 108 and/or the fins 110A and 110B can be formed (with or without one another and with or without a remainder of the tibial baseplate 100) by additive manufacturing such by laser sintering or the like.

Similarly, referring to FIG. 1H, features such as the pegs 122A, 122B, the reference features 124A, 124B and 124C, the windows 126 and the porous or highly porous material 128 can be monolithically or integrally formed with the base B such as by additive manufacturing or other processing.

FIG. 1L shows an enlargement of the reference feature 124A of FIG. 1H. The reference feature 124A can have a distal end that forms part of the distal surface 104. The reference feature 124A can extend generally distal from the base B and can be formed of similar or identical material to the base B such as the relatively less porous or non-porous biocompatible material 150. This relatively less porous or non-porous biocompatible material 150 can be joined with the porous or highly porous material 128 using laser sintering, a bonding layer or other technique as known in the art.

Described another way, the porous or highly porous material 128 can form at least a first layer 152 that forms at least a majority of the distal surface 104. The relatively less porous or non-porous biocompatible material 150 can comprise at least a second layer 154. The reference feature 124A can extend through the first layer 152 and can form at least a portion of the distal surface 104.

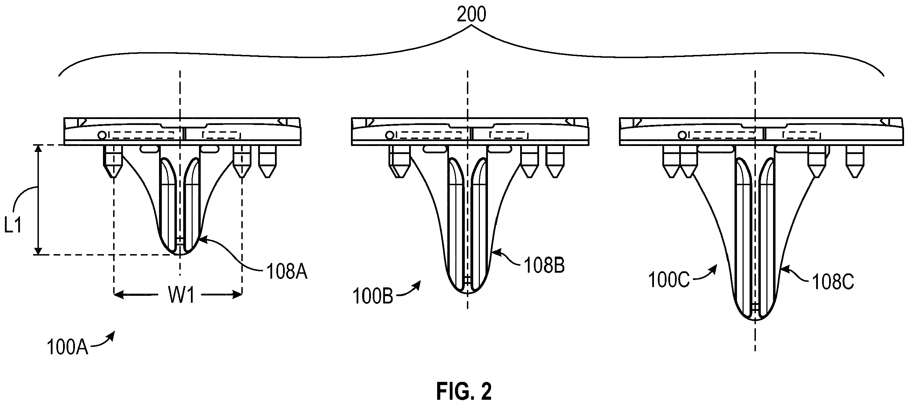

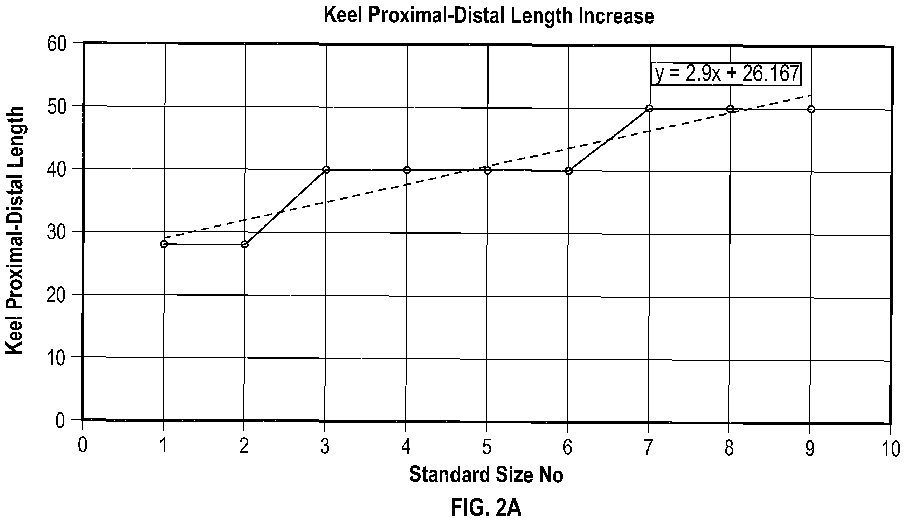

FIG. 2 shows a system 200 that includes various sizes of tibial baseplates 100A, 100B and 100C. The tibial baseplate 100A can be a standard size 1 configured for a patient with a small statured knee. The tibial baseplate 100B can be a standard size 5 configured for a patient with a middle statured knee. The tibial baseplate 100C can be a standard size 8 configured for a patient with a larger statured knee. The tibial baseplates 100A, 100B and 100C can be configured to provide a desired amount of coverage to the resected tibia without overhang or other undesired mounting arrangement. The tibial baseplates 100A, 100B and 100C can differ in regards to dimensions such as a medial-lateral extent (e.g. as measured from a first medial most edge of the periphery to a second lateral most edge), anterior-posterior extent (e.g. as measured from a third anterior most edge of the periphery to a fourth posterior most edge). However, a medial-lateral width W1 of the tibial baseplate 100A may only vary from that of the tibial baseplate 100B or tibial baseplate 100C by 20 mm or less, for example. Thus, the medial-lateral width W1 may not grow with changes in standard size in a manner commiserate with other dimensions (e.g., the medial-lateral extent of the periphery, the anterior-posterior extent of the periphery, etc.). A proximal-distal length L1 of the tibial baseplate 100A may only vary from that of the tibial baseplate 100B or tibial baseplate 100C by a substantial amount as shown in FIGS. 2 and 2A.

FIG. 2 shows the tibial baseplates 100A, 100B and 100C are all non-stemmable in construction (although stemmable constructs are contemplated). Thus, a proximal-distal length of the keel 108A increases generally with an increase in standard size. Thus, the proximal distal length of the keel 108A of the tibial baseplate 100A is relatively shorter in extent than that of the keel 108B and the keel 108C. FIG. 2A shows the increase in the proximal-distal length of the keel 108A can be step-wise increase with two or more of the different standard sizes each sharing a same proximal-distal length for the family of standard sizes. Put another way, the proximal-distal length does not increase linearly in a straight line manner with increase in a standard size but increases in a step-wise manner. Thus, at least two of the standard sizes (and sometimes up to four of the standard sizes) share the same proximal-distal length.

Exemplary lengths for the keel per standard size are provided in FIG. 2A. It should be noted the keel length does not smoothly transition in a straight line manner but has a step-functionality where several sizes (sizes 1-2, sizes-3-6 and sizes 7-9) can share substantially a same proximal-distal length. However, the general trend as shown by best fit curve is a linear increase in the proximal-distal length of the keel with change in size. It should be noted that other features discussed herein such as angles of flutes, flute radius, flute medial-lateral width, anterior fin size (medial-lateral width) and/or fin radius, posterior flute angle (e.g. angle between the fins), distal tip of keel geometry, etc. can remain substantially constant in geometry with changes in the standard size. Thus, for example a standard size 1 tibial baseplate 100A will have the same size (medial-lateral) anterior grooves as that of a standard size 8 tibial baseplate 100C (see FIG. 2).

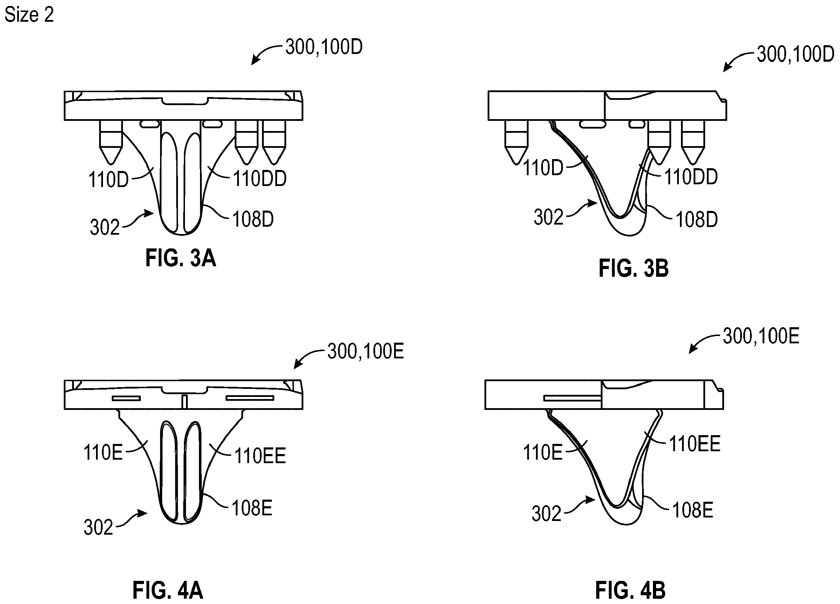

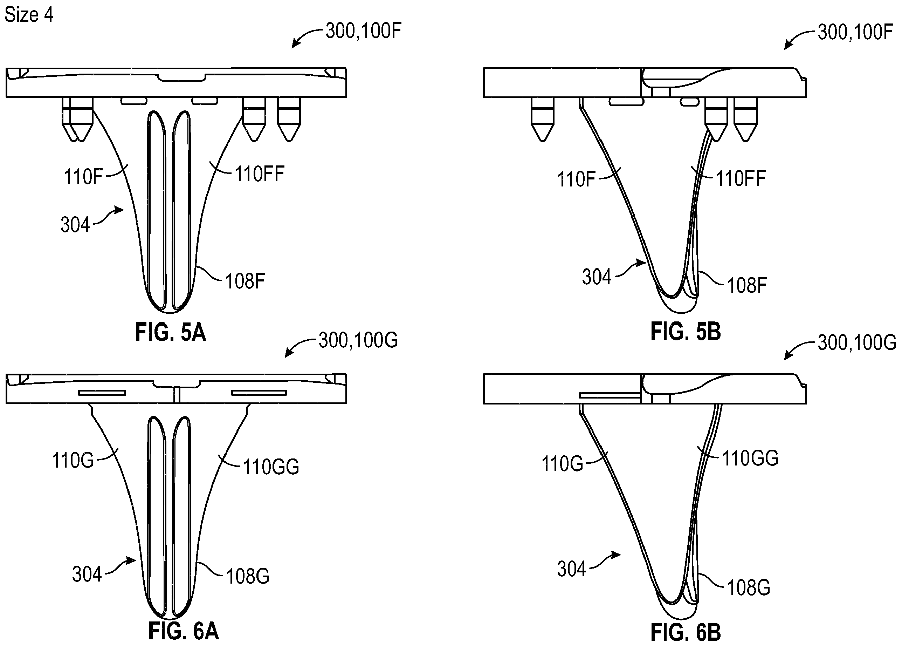

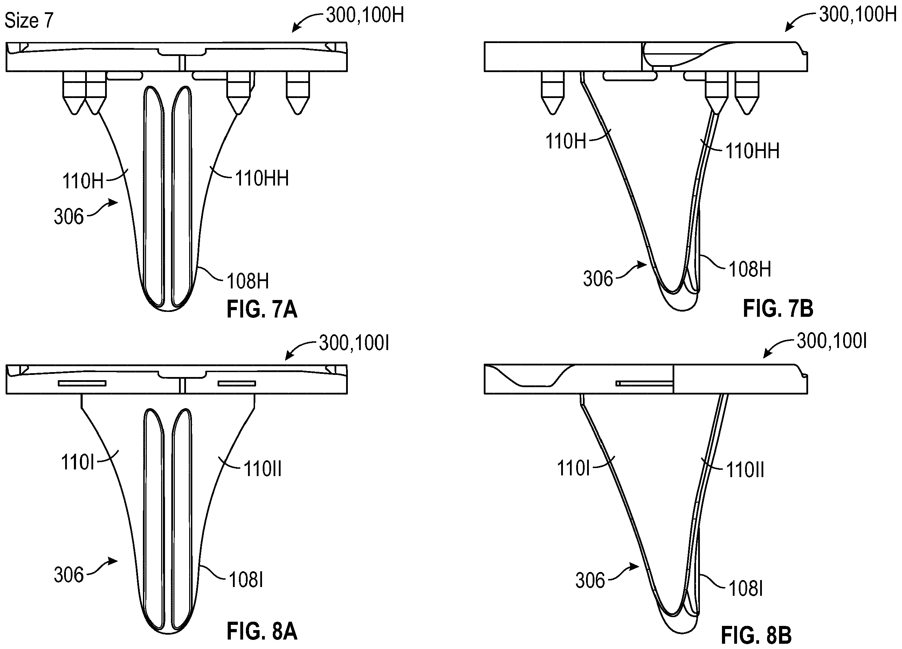

FIGS. 3A-8B show another aspect of a system 300 of tibial baseplates. In particular, the system 300 includes, for example, tibial baseplate 100D (FIGS. 3A and 3B), tibial baseplate 100E (FIGS. 4A and 4B), tibial baseplate 100F (FIGS. 5A and 5B), tibial baseplate 100G (FIGS. 6A and 6B), tibial baseplate 100H (FIGS. 7A and 7B) and tibial baseplate 100I (FIGS. 8A and 8B).

The tibial baseplates 100D and 100E can be of a same standard size (e.g., a standard size 2) but of a different construction in that the tibial baseplate 100D is configured for uncemented fixation (rather utilizing bone ingrowth) as compared with the tibial baseplate 100E, which is configured for cemented fixation.
Similarly, the tibial baseplates 100F and 100G can be of a same standard size (e.g., a standard size 4) but of a different construction in that the tibial baseplate 100F is configured for uncemented fixation (rather utilizing bone ingrowth) as compared with the tibial baseplate 100G, which is configured for cemented fixation. The tibial baseplates 100H and 100I can be of a same standard size (e.g., a standard size 7) but of a different construction in that the tibial baseplate 100H is configured for uncemented fixation (rather utilizing bone ingrowth) as compared with the tibial baseplate 100I, which is configured for cemented fixation.

The tibial baseplates 100D and 100E can share substantially a same size (e.g., the medial-lateral extent of the periphery can be substantially the same, the anterior-posterior extent of the periphery can be substantially the same, etc.). Additionally, the geometry of the tibial baseplates 100D and 100E distal features can also be substantially the same. This includes geometry of a plurality of aspects for the keel 108D and the fins 110D, 110DD of the tibial baseplate 100D as compared with corresponding aspects for the keel 108E and the fins 110E, 110EE of the tibial baseplate 100E. Thus, as shown in FIGS. 3A-4B, the keel 108D and the fins 110D, 110DD of the tibial baseplate 100D can share substantially a same distal profile 302 (as measured along distal-most surfaces of the keel 108D and the fins 110D, 110DD moving medial-lateral along an extent of the keel 108D and the fins 110D, 110DD) as the keel 108E and the fins 110E, 110EE of the tibial baseplate 100E. Put another way, the keels 108D and 108E can share same geometric features such as but not limited to proximal-distal length, diameter, groove shape and size, flute size and shape, thickness, etc. Similarly, the fins 110D, 110DD can share a same geometry (angulation, medial-lateral width, proximal-distal length, etc.) as the fins 110E, 110EE. Such substantial match in shapes for the keel 108D and the fins 110D, 110DD with the keel 108E and the fins 110E, 110EE can allow the surgeon to choose intraoperatively between a cemented apparatus or a non-cemented apparatus. In this manner, reaming or other surgical steps that would otherwise need to be carried out to switch from a cemented apparatus to a non-cemented apparatus (or vice versa) need not be performed saving time and complexity. According to some examples, the fins 110D, 110DD and the fins 110E, 110EE may only differ in a thickness of the fins 110D, 110DD may be slightly greater than a corresponding thickness of the fins 110E, 110EE.

In the manner discussed with the tibial baseplates 100D and 100E, the tibial baseplates 100F and 100G can share substantially a same size (e.g., the medial-lateral extent of the periphery can be substantially the same, the anterior-posterior extent of the periphery can be substantially the same, etc.). Additionally, the geometry of the tibial baseplates 100F and 100G distal features can also be substantially the same. This includes geometry of a plurality of aspects for the keel 108F and the fins 110F, 110FF of the tibial baseplate 100F as compared with corresponding aspects for the keel 108G and the fins 110G, 110GG of the tibial baseplate 100G. Thus, as shown in FIGS. 5A-6B, the keel 108F and the fins 110F, 110FF of the tibial baseplate 100F can share substantially a same distal profile 304 (as measured along distal-most surfaces of the keel 108F and the fins 110F, 110FF moving medial-lateral along the extent of the keel 108F and the fins 110F, 110FF) as the keel 108G and the fins 110G, 110GG of the tibial baseplate 100G. Put another way, the keels 108F and 108G can share same geometric features such as but not limited to proximal-distal length, diameter, groove shape and size, flute size and shape, etc. Similarly, the fins 110F, 110FF can share a same geometry (angulation, medial-lateral width, proximal-distal length, thickness, etc.) as the fins 110G, 110GG. According to some examples, the fins 110F, 110FF and the fins 110G, 110GG may only differ in a thickness of the fins 110F, 110FF may be slightly greater than a corresponding thickness of the fins 110G, 110GG.

The tibial baseplates 100H and 100I can share substantially a same size (e.g., the medial-lateral extent of the periphery can be substantially the same, the anterior-posterior extent of the periphery can be substantially the same, etc.). Additionally, the geometry of the tibial baseplates 100H and 100I distal features can also be substantially the same. This includes geometry of a plurality of aspects for the keel 108H and the fins 110H, 110HH of the tibial baseplate 100H as compared with corresponding aspects for the keel 108I and the fins 110I, 110II of the tibial baseplate 100I. Thus, as shown in FIGS. 7A-8B, the keel 108H and the fins 110H, 110HH of the tibial baseplate 100H can share substantially a same distal profile 306 (as measured along distal-most surfaces of the keel 108H and the fins 110H, 110HH moving medial-lateral along the extent of the keel 108H and the fins 110H, 110HH) as the keel 108I and the fins 110I, 110II of the tibial baseplate 100I. Put another way, the keels 108H and 108I can share a same geometry of features such as but not limited to proximal-distal length, diameter, groove shape and size, flute size and shape, etc. Similarly, the fins 110H, 110HH can share a same geometry (angulation, medial-lateral width, proximal-distal length, thickness, etc.) as the fins 110I, 110II. According to some examples, the fins 110H, 110HH and the fins 110I, 110II may only differ in a thickness of the fins 110H, 110HH may be slightly greater than a corresponding thickness of the fins 110I, 110II.

According to the examples provided herein, the tibial baseplate can comprise a cruciate retaining (CR) design. Thus, the tibial baseplates can have a relieve for the posterior cruciate ligament is not resected during implantation. However, other prosthesis designs are contemplated including a posterior-stabilized (PS) design, a mid-level constraint (MLC) or constrained posterior stabilized (CPS) design, and an ultra-congruent (UC) design, for example. The PS and MLC designs utilize a spine and cam as known in the art. The posterior cruciate ligament is eliminated so no relief need be provided.

### Additional Notes

The above detailed description includes references to the accompanying drawings, which form a part of the detailed description. The drawings show, by way of illustration, specific embodiments in which the invention can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "generally" "substantially" "about" mean within 15 percent of the value provided (±). The terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects.

The above description is intended to be illustrative, and not restrictive. For example, the above-described examples (or one or more aspects thereof) can be used in combination with each other. Other examples can be used, such as by one of ordinary skill in the art upon reviewing the above description. The Abstract is provided to comply with 37 C.F.R. §1.72(b), to allow the reader to quickly ascertain the nature of the technical disclosure. It is submitted with the understanding that it will not be used to interpret or limit the scope or meaning of the claims. Also, in the above detailed description, various features can be grouped together to streamline the disclosure. This should not be interpreted as intending that an unclaimed disclosed feature is essential to any claim. Rather, inventive subject matter can lie in less than all features of a particular disclosed example. Thus, the following claims are hereby incorporated into the detailed description as examples or embodiments, with each claim standing on its own as a separate example, and it is contemplated that such examples can be combined with each other in various combinations or permutations. The scope of the invention should be determined with reference to the appended claims, along with the full scope of equivalents to which such claims are entitled.

## Claims

1. A tibial prosthesis for a knee arthroplasty comprising:
a baseplate comprising:
a distal surface sized and shaped to substantially cover a proximal resected surface of a tibia;
a proximal surface opposite the distal surface, the proximal surface having a lateral compartment and a medial compartment opposite the lateral compartment;
a periphery extending between the distal surface and the proximal surface;
a first layer of porous material forming at least a majority of the distal surface and extending to the periphery; and
a second layer of non-porous or relatively less porous material having a plurality of reference features extending through the first layer, wherein the plurality of reference features form at least a portion of the distal surface; and
a tibial keel extending distally from the distal surface to define a longitudinal tibial keel axis.

2. The tibial prosthesis of claim 1, further a plurality of fins spanning a junction between the tibial keel and the distal surface.

3. The tibial prosthesis of claim 2, wherein the plurality of fins are angled anterior-posterior and medial-lateral to form a flute on a posterior of the tibial keel.

4. The tibial prosthesis of claim 3, wherein the tibial keel includes a second flute and a third flute each extending to a distal tip of the tibial keel, wherein the second flute and the third flute are connected by a anterior fin on an anterior of the tibial keel.

5. The tibial prosthesis of any one of claims 2-4, further comprising a plurality of pegs extending distally from the distal surface, wherein one or both of the plurality of pegs or the plurality of fins has a window formed of the porous material therein.

6. The tibial prosthesis of claim 5, wherein the plurality of pegs comprises four pegs located adjacent, respectively, a posterior-medial corner, a posterior-lateral corner, an anterior-lateral corner and an anterior-medial corner of the periphery.

7. The tibial prosthesis of any one of claims 5-6, wherein the plurality of pegs are one of square shaped or rectangular in cross-section and are configured to be press-fit into a round hole drilled into the tibia.

8. The tibial prosthesis of any one claims 5-7, wherein the window extends entirely through at least one of the plurality of fins.

9. The tibial prosthesis of any one of claims 2-8, wherein one or more surfaces of at least one of the tibial keel or the plurality of fins is fabricated to be smoother than the distal surface to discourage bone ingrowth.

10. A tibial prosthesis system comprising:
a plurality of prostheses, each of the plurality of prostheses including:
a baseplate comprising:
a distal surface sized and shaped to substantially cover a proximal resected surface of a tibia;
a proximal surface opposite the distal surface, the proximal surface having a lateral compartment and a medial compartment opposite the lateral compartment;
a periphery extending between the distal surface and the proximal surface; and
a plurality of pegs extending distally from the distal surface;
wherein a size of each of the plurality of prostheses differ with respect to the distal surface, the proximal surface and the periphery; and
a tibial keel extending distally from the distal surface to a dome shaped distal tip, wherein the tibial keel has an elongate length as measured proximal-distal and defines a longitudinal tibial keel axis extending along the elongate length, wherein the elongate length increases in a step-wise manner with an increase in the size the plurality of prostheses with respect to the distal surface, the proximal surface and the periphery.

11. The system of claim 10, further a plurality of fins spanning a junction between the tibial keel and the distal surface.

12. The system of claim 11, further comprising:
a first layer of porous material forming at least a majority of the distal surface and extending to the periphery; and
a second layer of non-porous or relatively less porous material forming a base;
wherein the plurality of pegs are formed of both the porous material and the non-porous or relatively less porous material.

13. The system of claim 12, wherein the tibial keel includes a first flute on a posterior side formed between the plurality of fins in addition to the tibial keel, and wherein the tibial keel includes a second flute and a third flute each extending to a distal tip of the tibial keel, wherein the second flute and the third flute are connected by a anterior fin on an anterior of the tibial keel.

14. The system of any one of claims 12-13, wherein the second layer of the non-porous or relatively less porous material has a plurality of reference features extending through the first layer, and wherein the plurality of reference features form at least a portion of the distal surface.

15. The system of any one of claims 10-14, wherein one or more surfaces of at least one of the tibial keel or the plurality of fins is fabricated to be smoother than the distal surface to discourage bone ingrowth.
